# EUROPÄISCHE PATENTSCHRIFT

(11) **EP 1 528 922 B1**
(45) Veröffentlichungstag und Bekanntmachung des Hinweises auf die Patenterteilung: **01.03.2006**
(21) Anmeldenummer: 03787778.4
(22) Anmeldetag: 12.08.2003
(51) Int. Cl.: A61K 31/21, A61P 25/00

(54) **VERWENDUNG VON TREOSULFAN UND DERIVATEN DAVON ZUR BEHANDLUNG DER MULTIPLEN SKLEROSE**
USE OF TREOSULFAN AND DERIVATIVES THEREOF FOR TREATING MULTIPLE SCLEROSIS
UTILISATION DE TREOSULFAN ET DE SES DERIVES POUR TRAITER LA SCLEROSE EN PLAQUES

(30) Priorität: 13.08.2002 DE 10237146
(43) Veröffentlichungstag der Anmeldung: 11.05.2005
(73) Patentinhaber: Medac Gesellschaft für klinische Spezialpräparate mbH, D-20354 Hamburg (DE)
(72) Erfinder: SASS, Gretel, 22083 Hamburg (DE)
(74) Vertreter: UEXKÜLL & STOLBERG
(86) Internationale Anmeldenummer: PCT/EP2003/008957
(87) Internationale Veröffentlichungsnummer: WO 2004/016263

(56) Entgegenhaltungen:
- WO-A-01/32154
- OPENSHAW H, LUND B, KASHYAP A, ATKINSON R, SNIECINSKI I, WEINER L, FORMAN S: "Peripheral blood stem cell transplantation in multiple sclerosis with busulfan and cyclophosphamide conditioning: Report of toxicity and immunological monitoring" BIOLOGY OF BLOOD AND MARROW TRANSPLANTATION, Bd. 6, Nr. 5a, 2000, Seiten 563-575, XP002263510

## Beschreibung

Die vorliegende Erfindung betrifft die Verwendung von Treosulfan und Derivaten davon zur Herstellung eines Arzneimittels zur Behandlung der Multiplen Sklerose.

Die Multiple Sklerose (MS) ist eine neurologische Erkrankung, die weltweit mehr als eine Million Menschen betrifft. Bei der MS handelt es sich um einen entzündlichen Zustand, der das Myelin des zentralen Nervensystems (ZNS) zerstört und neurologische Beeinträchtigungen sowie häufig auch schwere Behinderungen verursacht. Die Ätiologie der MS ist bislang unbekannt, wobei im allgemeinen angenommen wird, daß die Erkrankung durch eine Art Autoimmunprozeß vermittelt wird, der möglicherweise durch eine Infektion ausgelöst und von einer genetischen Prädisposition überlagert wird. Nach meist schleichendem, selten subakutem oder akutem Beginn im 20. bis 40. Lebensjahr wird am häufigsten ein chronisch-progredienter oder schubweiser Verlauf mit Neigung zu Remissionen beobachtet. Die unterschiedlichen Stadien und Formen der Multiplen Sklerose werden unterteilt in: Schubförmigremittierend (relapsing-remitting; RR) MS (derzeit etwa 80-85% der MS-Patienten), primär-progressive (PP) MS, wobei mit der Zeit mehr als 50% der Patienten mit RR-MS schließlich eine bleibende Verschlechterung mit oder ohne überlagerte Rückfälle entwickelt (sekundär-progressive (SP) Form der MS). Es ist unklar, ob die verschiedenen Krankheitsverläufe auf den selben oder unterschiedlichen pathophysiologischen Prozessen beruhen.

Zur Behandlung der unterschiedlichen Stadien und Formen der MS gibt es zum Teil eindeutige Behandlungsempfehlungen, die sich in einer immunmodulatorischen Stufentherapie der MS widerspiegeln (P. Rieckmann, "Immunmodulatorische Therapie der Multiplen Sklerose: Konsensusprotokolle im deutschsprachigen Raum und Nordamerika" in: "Multiple Sklerose: Kausalorientierte, symptomatische und rehabilitative Therapie", Springer Verlag Berlin - Heidelberg - New York 2002: 109-118). Als Schubtherapie haben sich Cortikosteroide bewährt. Für die schubförmige remittierende Multiple Sklerose stehen mit Interferon β und Glatirameracetat immunologisch wirksame Substanzen zur Verfügung, die die Anzahl der Schübe reduzieren. Interferon β ist ebenfalls wirksam bei der sekundären progressiven MS, sofern in diesem Stadium noch Schübe vorhanden sind.

Für die Therapie der rein progressiven MS stehen keine eindeutig als wirksam belegten Arzneimittel zur Verfügung. Sowohl Mitoxantron als auch Cyclophosphamid oder Methotrexat kommen zur Anwendung. Gemäß der Behandlungsrichtlinie der MS Council for Clinical Practice Guidelines von 2002 (D.S. Goodin et al., Neurology 58 (2002) 169-178) wird die Effektivität dieser Substanzen als möglich angesehen. Da bei Mitoxantron ab einer kumulativen Dosierung von 160 mg/m² kardiale Nebenwirkungen auftreten (Fachinformation "Novantron®" (Wyeth Pharma GmbH), Januar 2002, Bundesverband der Pharmazeutischen Industrie e.V./FachInfo-Service, D-88322 Aulendorf), weil bei Cyclophosphamid unter anderem Schäden der ableitenden Harnwege möglich sind und Methotrexat häufig gastrointestinale Nebenwirkungen aufweist, werden nebenwirkungsärmere mindestens äquieffektive Alternativen benötigt.

Im Hinblick auf die noch immer bestehenden Probleme bei der Suche nach geeigneten Wirkstoffen zur Behandlung von MS ist es daher Aufgabe der vorliegenden Erfindung, ein Arzneimittel bereitzustellen, das zur Behandlung von MS (einschließlich aller Verlaufsformen) geeignet ist und das die bei den bislang eingesetzten Wirkstoffen bekannten Nachteile nicht aufweist.

Die Aufgabe wird erfindungsgemäß durch die Verwendung von Treosulfan oder Derivaten davon gelöst.

Es hat sich im Rahmen der vorliegenden Erfindung überraschenderweise gezeigt, dass Treosulfan nicht nur außerordentlich gut verträglich ist, sondern bei MS-Patienten zu einer deutlichen Verbesserung des Krankheitsverlaufs geführt hat.

Am Beispiel der experimentellen autoimmunen Enzephalomyelitis (EAE), die als Tiermodell der menschlichen MS anerkannt ist, wurde zunächst die Wirksamkeit von Treosulfan überprüft. Die Effektivität der Treosulfan-Behandlung wurde im Vergleich zu einer unbehandelten Kontrollgruppe und einer Gruppe, die Mitoxantron verabreicht bekam, untersucht. In ersten Versuchen wurde Treosulfan am Tag der Immunisierung appliziert, in einem weiteren an Tag 14 nach Immunisierung. Die bis zu diesem Tag manifesten Schäden konnten zwar nicht rückgängig gemacht werden, im Gegensatz zur (unbehandelten) Kontrollgruppe, bei der die Erkrankung weiter fortschritt, war bei der mit Treosulfan behandelten Gruppe eine Stabilisierung zu beobachten. Bei zahlreichen Versuchstieren trat allerdings eine auffällige Besserung ein. Bemerkenswert war ferner, daß in der Treosulfan-Gruppe 7 von 8 Tieren an Tag 53 noch lebten, während in der Vergleichsgruppe lediglich 2 von 8 Tieren zu diesem Zeitpunkt noch am Leben waren. In der Vergleichsgruppe, die Mitoxantron erhielt, wurde keine bessere Effektivität beobachtet als in der Treosulfan-Gruppe.

Bei Treosulfan handelt es sich um (2S,3S)-Threitol-1,4-bismethansulfonat (L-Threitol-1,4-bis(methansulfonat); Chemical Abstracts Registry No. 299-75-2):

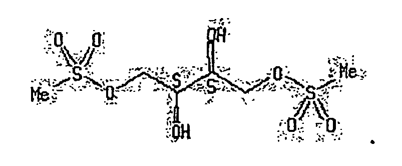

Erfindungsgemäß sind auch Derivate von Treosulfan eingeschlossen, wie z.B. Busulfan ((1,4-Bis(methylsulfonyloxy)-butan), Dimethylbusulfan (1,4-Bis(methylsulfonyloxy)-1,4-dimethylbutan; CA Registry No. 55-93-6), Pentasulfan (1,5-Dimesyloxypentan; CA Registry No. 2374-22-3), Hepsulfam (1,7-Heptanedioldisulfamat; CA Registry No. 96892-57-8) oder ähnliche Substanzen, die in ersten Kontrollversuchen zu ähnlichen Ergebnissen geführt haben wie Treosulfan. In Betracht kommen auch Treosulfan-Derivate, bei denen die Methylgruppen am Schwefelatom ausgetauscht (beispielsweise durch (niedere) Alkylsubstituenten (linear oder verzweigt), insbesondere mit 1 bis 7 C-Atomen, wie z.B. Ethyl-, Propyl- Butyl-, Pentyl-, Hexyl-und Heptyl- usw.) oder substituiert (Ersatz eines/mehrerer Wasserstoffatome durch einen/mehrere Substituenten, wie z.B. Isopropyl, Tertiärbutyl sind.

Busulfan wurde bislang ausschließlich zur Konditionierung von Patienten vor Stammzelltransplantation eingesetzt (vgl. Openshaw et al., Biology of Blood and Marrow Transplantation 2000, 6:563-575), mit dem Ziel, das Anwachsen des Transplantats zu fördern. Demgegenüber wurde im Rahmen der vorliegenden Erfindung überraschenderweise gefunden, daß sich Treosulfan und/oder dessen Derivate, einschließlich Busulfan, direkt zur Behandlung von MS eignen. Gegenüber dem risikoreichen und aufwendigen Ansatz der Stammzelltransplantation stellt die Erfindung eine unabhängige und erfolgreiche Alternative dar.

Gegenstand der vorliegenden Erfindung ist daher die Verwendung von Treosulfan und Derivaten davon zur Herstellung einer pharmazeutischen Zusammensetzung zur Behandlung von MS. Als Derivate werden beispielsweise die oben genannten Substanzen eingesetzt. Erfindungsgemäß eingeschlossen sind ferner Kombinationen von Treosulfan und/oder einem Derivat davon mit einer oder mehreren immunmodulatorischen Substanzen, d.h. Kombinationspräparate, die neben Treosulfan und/oder einem Derivat davon eine oder mehrere immunmodulatorisch wirksame Substanzen, wie z.B. Interferon- (IFN-) und/oder Glatriameracetat, als weiteren Wirkstoff enthalten.

Die pharmazeutische Zusammensetzung liegt vorzugsweise in Form einer zur intravenösen Applikation (Infusion) geeigneten Lösung vor, es kann aber auch eine orale Formulierung in Betracht kommen.

Die Dosierung liegt bei 1 bis 10 g, vorzugsweise 3 bis 9 g und besonders bevorzugt 5 bis 8 g Treosulfan (und/oder Treosulfan-Derivat) pro m² Körperoberfläche.

Die pharmazeutische zusammensetzung dient zur Behandlung der multiplen Sklerose, einschließlich der Behandlung der schubförmig-remittierenden, der primär progressiven und insbesondere der sekundär progressiven MS.

Die vorliegende Erfindung wird nachfolgend anhand von Beispielen näher erläutert.

### Beispiele

### 1. Vorarbeiten

Es wurden zunächst mehrere Versuche zur Dosisfindung von Treosulfan in DA-Ratten, einem speziellen für diese Versuche geeigneten Rattenstamm (Stamm Dark-Agouti), mit Myelin-Oligodendrozyten-Glykoprotein (MOG)-induzierter experimenteller autoimmuner Enzephalomyelitis (EAE) durchgeführt. Diese Ratten stellen ein sehr gutes Modell für die Multiple Sklerose (MS) dar, da der klinische Verlauf und die Läsionsmorphologie (Entzündung, Entmarkung, Verlust von Axonen und Nervenzellen) der MS reproduziert werden können.

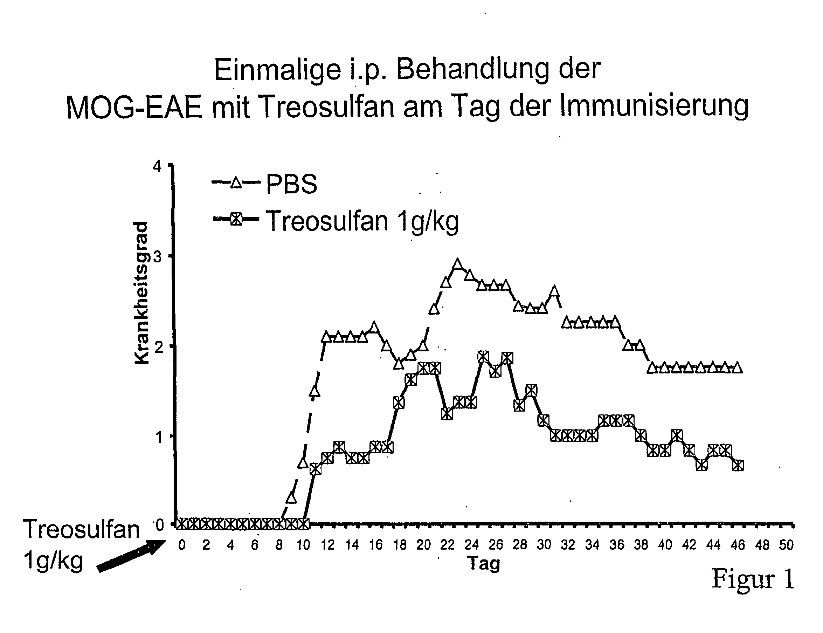

Es zeigte sich eine einmalige intraperitoneal verabreichte Dosis von 1 g/kg Körpergewicht als verträglich und fähig, die Krankheit zu unterdrücken (siehe Figur 1). Die injizierten Tiere zeigten zu Beginn der Behandlung lediglich eine deutliche Gewichtsabnahme.

### 2. Untersuchung der Treosulfan-Wirkung auf die MOG-EAE

### 2.1 Klinische Charakterisierung

DA-Ratten (10 Tiere je Gruppe) wurden entweder mit einer einmaligen Dosis Treosulfan (0,5 g/kg Körpergewicht) am Tag der Immunisierung (p.i.) behandelt oder mit einer dreimaligen Treosulfan-Applikation (0,5 g/kg Körpergewicht je Applikation) im Abstand von 1 Tag. Als Kontrolle wurde PBS (Phosphatgepufferte Salzlösung) verabreicht. In einem weiteren Experiment wurde Treosulfan (einmalige Dosis: 0,5 g/kg Körpergewicht) 2 Wochen nach erfolgter Immunisierung verabreicht. Die Gesamtzahl der Ratten betrug 30 Tiere. Die Tiere wurden für die Dauer von 40 Tagen nach Initiierung des Experiments klinisch beobachtet und regelmäßig neurologisch untersucht.

### 2.2 Histopathologische Untersuchung der Läsionen

Am Tag 40 wurden die Ratten perfundiert und das zentrale Nervensystem (ZNS) histopathologisch analysiert (im Hinblick auf Entzündung, Entmarkung und Axonverlust). (R. Weissert et al., J. Clin. Invest. 102 (1998), 1265 - 1273).

### 2.3 Immunologische Charakterisierung

In einem zweiten Experiment wurden jeweils fünf Ratten am Tag 12 und Tag 40 (Gesamtzahl der Tiere: n = 30) nach Immunisierung immunologisch in bezug auf T- und B-Zell-Antworten charakterisiert (EliSpot (Autoimmun Diagnostika GmbH, D-72479 Strassberg) für IFN- sezernierende Zellen, Proliferation, Auto-Antikörperanalyse). (R. Weissert et al., J. Immunol. 160 (1989) 681 - 690).

### 2.4 Hämatologische Charakterisierung

Zur Analyse des hämatologischen Status wurde den Ratten am Tag 0, Tag 5, Tag 14 und Tag 53 nach Immunisierung Blut entnommen und eine Bestimmung von Hämoglobin (Hb), Retikulozyten, Leukozyten, Thrombozyten und Erythrozyten durchgeführt.

### 2.5 Ergebnisse

Die oben genannten Daten wurden zur Absicherung präliminarer Daten aus Dosis-Findungsversuchen mit einer aussagefähigen Anzahl an Tieren erhoben.

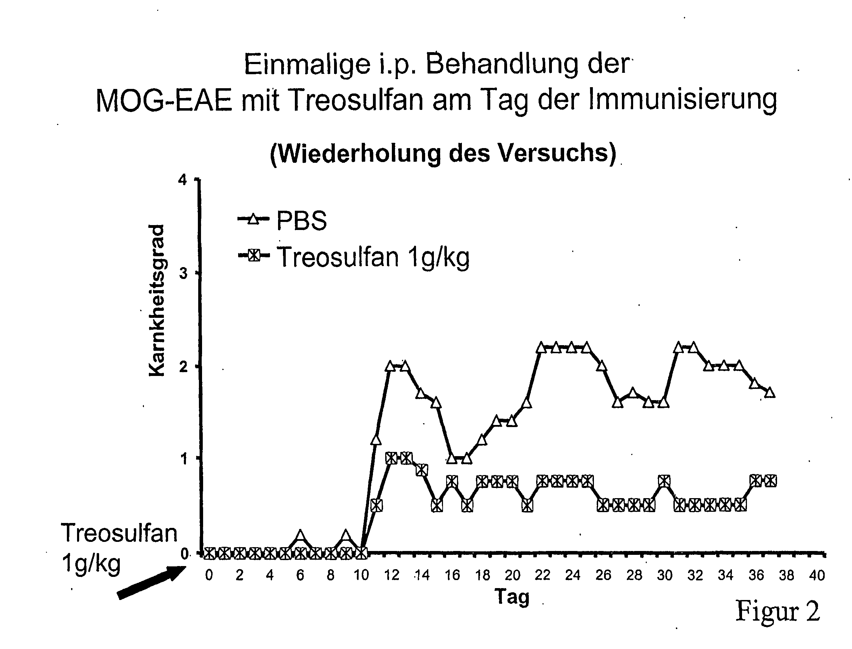

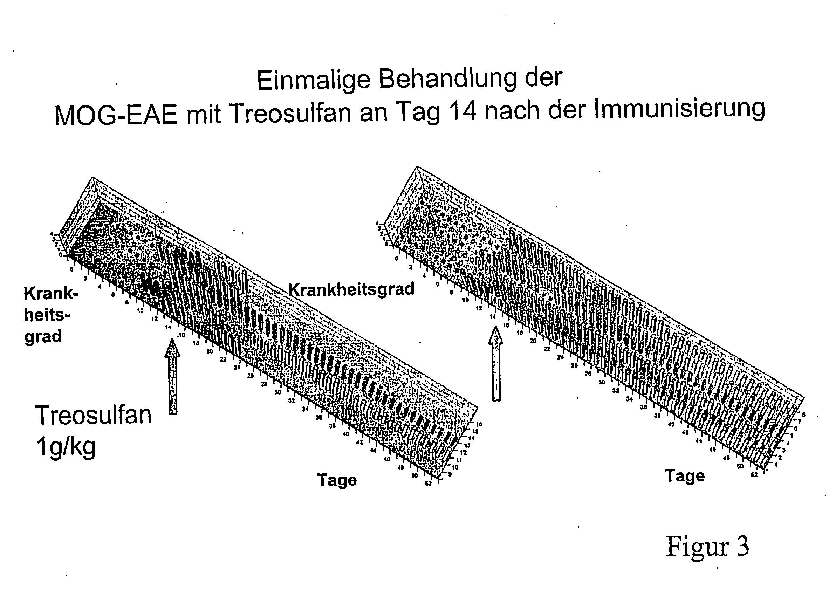

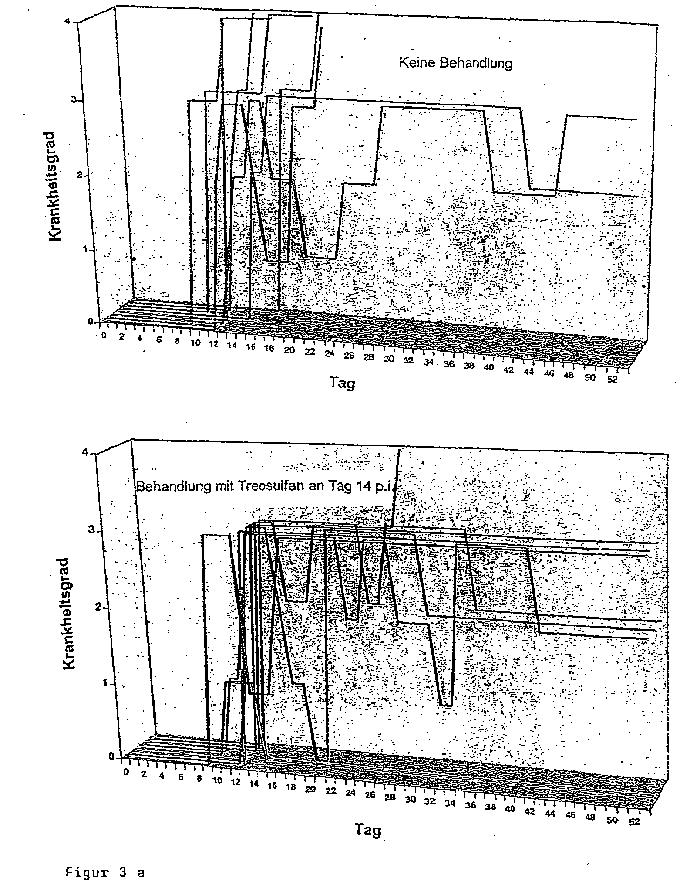

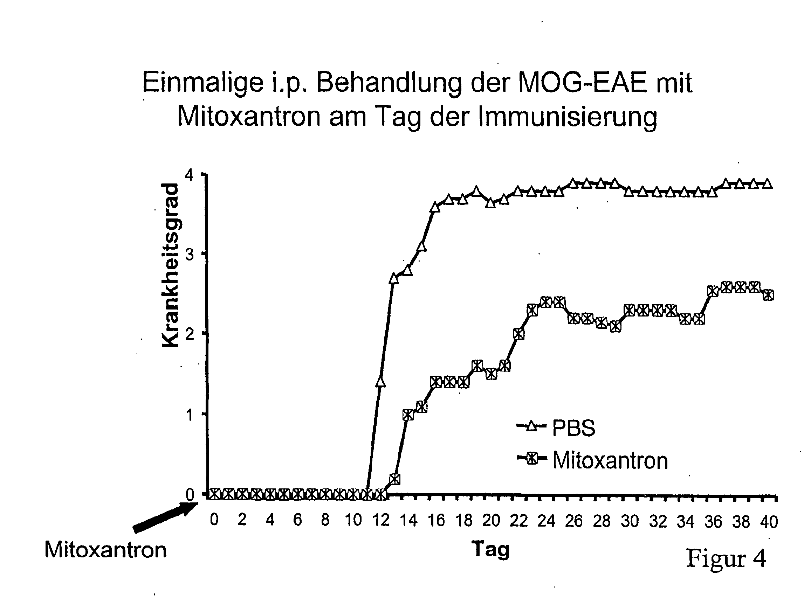

Die Ergebnisse sind in den Figuren 1 - 17 dargestellt. Die erhaltenen Ergebnisse belegen zunächst die Effektivität der Treosulfan Behandlung im Vergleich zu der unbehandelten Kontrollgruppe, die in den Figuren 1 - 3a dargestellt ist. Figur 4 zeigt die Effektivität von Mitoxantron unter den gleichen Versuchsbedingungen. Figur 3 bzw. 3a sind unterschiedliche Darstellungen des Experiments, in der Treosulfan an Tag 14 nach der Immunisierung verabreicht wurde. Die bis zu diesem Tag manifesten Schäden konnten naturgemäß nicht rückgängig gemacht werden. Im Gegensatz zur Kontrollgruppe, bei der die Erkrankung weiter fortschritt war allerdings bei der mit Treosulfan behandelten Gruppe eine Stabilisierung zu beobachten. Bei einigen Versuchstieren trat sogar eine auffällige Besserung ein.

Ferner wurde festgestellt, daß in der mit Treosulfan behandelten Gruppe 7 von 8 Tieren an Tag 53 noch lebten, während in der Vergleichsgruppe (unbehandelte Tiere) lediglich 2 von 8 Tieren noch am Leben waren.

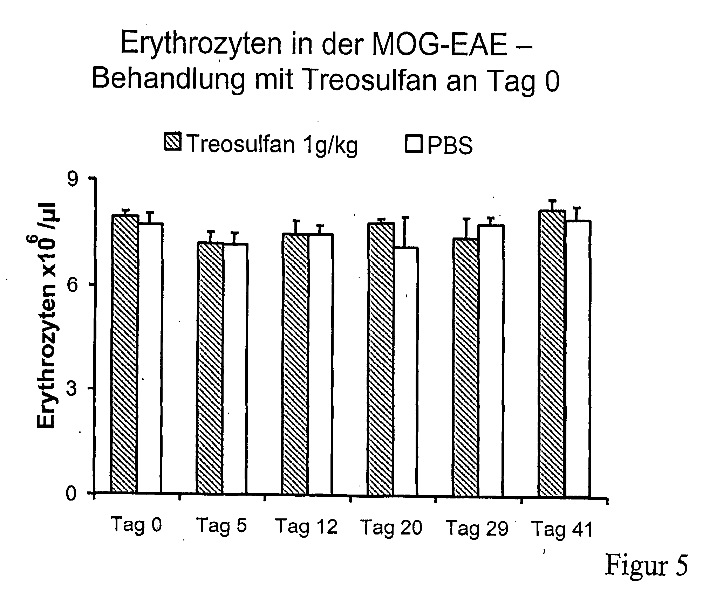

Die Verträglichkeit von Treosulfan ist in den Figuren 5 - 15 dargestellt. Veränderungen des Blutbildes werden nicht dokumentiert.

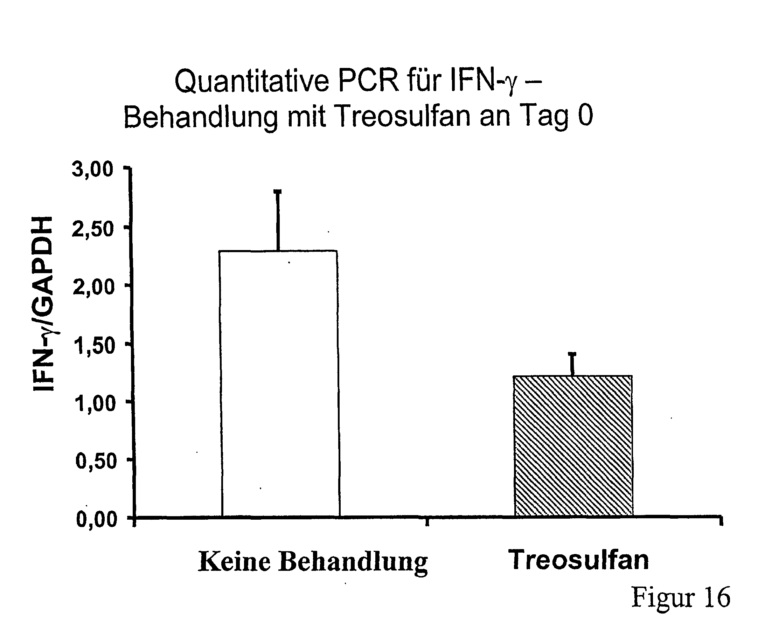

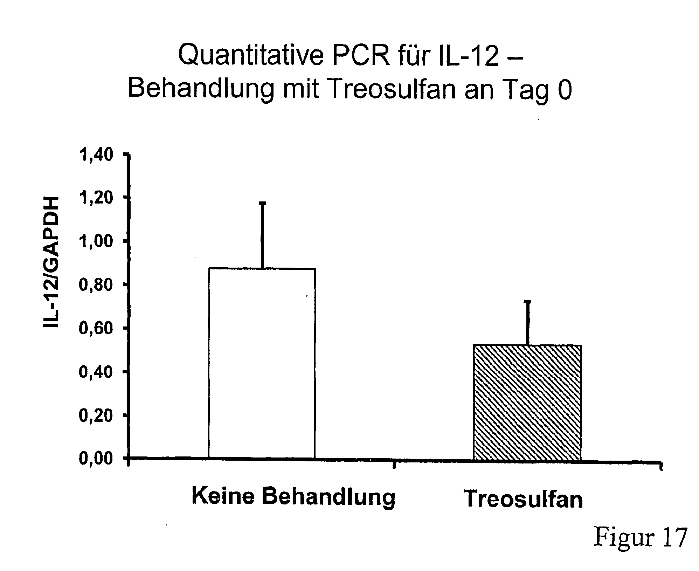

Beobachtet wurde ferner, dass die Interleukin 12 (IL-12) bzw. Interferon- (IFN-)-Werte in der Treosulfan-Gruppe erniedrigt waren (Figur 16, 17). (R. Weissert et al., J. Immunol. 166 (2001) 7588 - 7599).

### 3. Durchführung von Heilversuchen an MS-Patienten

In einem ersten Heilversuch wurden 5 Patienten mit sekundär progressiver MS (SPMS-Patienten) behandelt. Es handelte sich um 3 Männer im Alter von 51/58/62 Jahren und zwei Frauen im Alter von 39/41 Jahren.

Im Rahmen der Therapie wurde Treosulfan in Form einer zur intravenösen Applikation (Infusion) geeigneten Lösung verabreicht. Die Patienten wurden mit einer Dosierung von jeweils 5 g/m² Körperoberfläche im Abstand von 4 Wochen im ersten Quartal behandelt. Anschließend erfolgte pro Quartal eine jeweils einmalige Treosulfan Applikation (5 g/m² Treosulfan).

Bei zwei Patienten war eine Verbesserung des Ambulationsindexes (S.L. Hauser et al. N. Engl. J. Med. 308 (1983) 173 - 178) zu sehen. Substanzbezogene Nebenwirkungen wurden nicht beobachtet.

### Beschreibung der Figuren:

- Fig. 1:: Einmalige i.p. Behandlung der MOG-EAE mit Treosulfan am Tag der Immunisierung.
- Fig. 2:: Einmalige i.p. Behandlung der MOG-EAE mit Treosulfan am Tag der Immunisierung (Wiederholung des Versuchs).
- Fig. 3a:: Einmalige Behandlung der MOG-EAE mit Treosulfan an Tag 14 nach der Immunisierung - andere Darstellung des Experiments.
- Fig. 3:: Einmalige Behandlung der MOG-EAE mit Treosulfan an Tag 14 nach der Immunisierung.
- Fig. 4:: Einmalige i.p. Behandlung der MOG-EAE mit Mitoxantron am Tag der Immunisierung.
- Fig. 5:: Erythrozyten in der MOG-EAE-Behandlung mit Treosulfan an Tag 0.

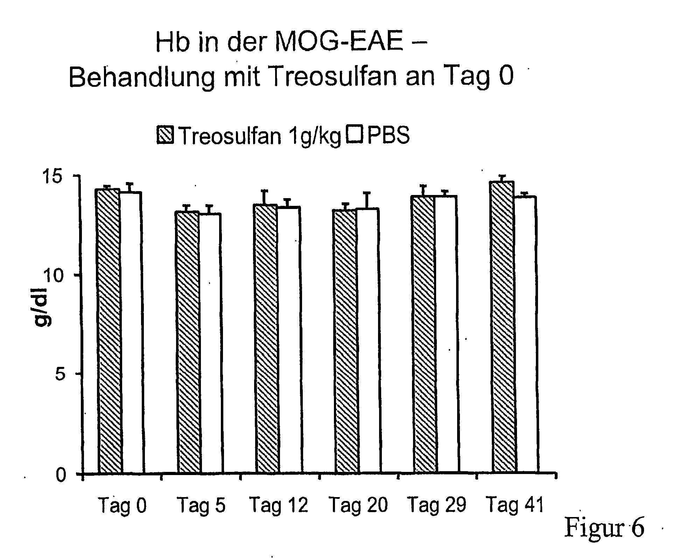

- Fig. 6:: Hämoglobin (Hb) in der MOG-EAE-Behandlung mit Treosulfan an Tag 0.

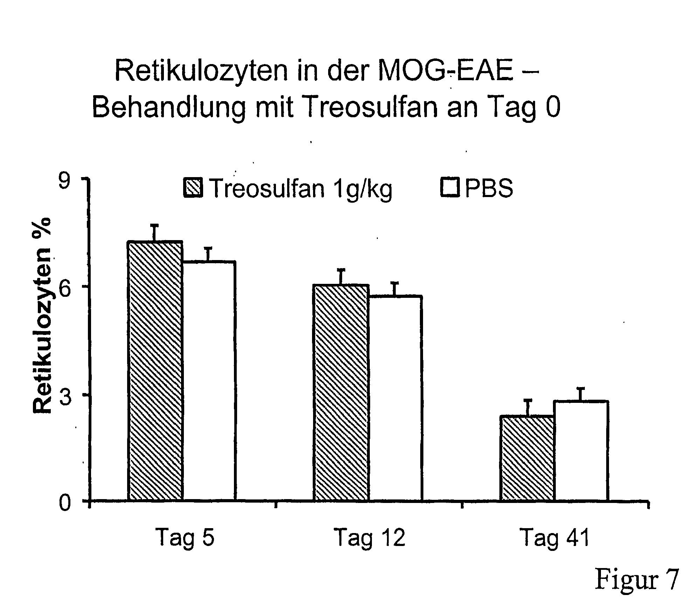

- Fig. 7:: Retikulozyten in der MOG-EAE-Behandlung mit Treosulfan an Tag 0.

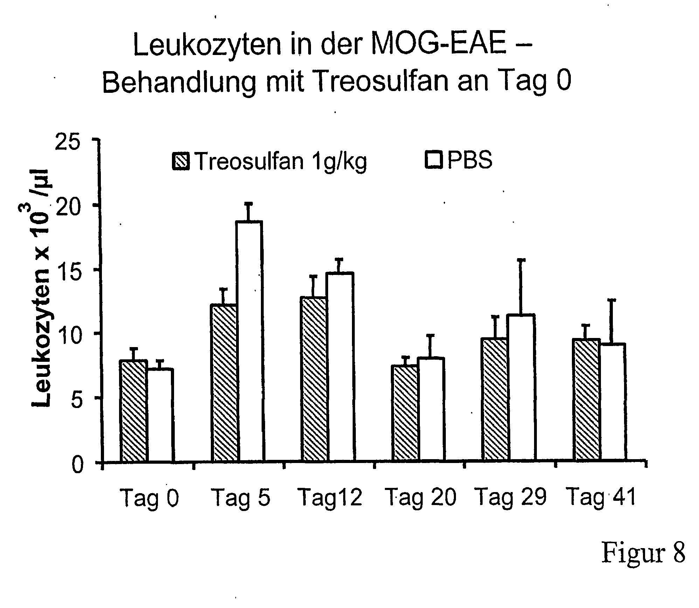

- Fig. 8:: Leukozyten in der MoG-EAE-Behandlung mit Treosulfan an Tag 0.

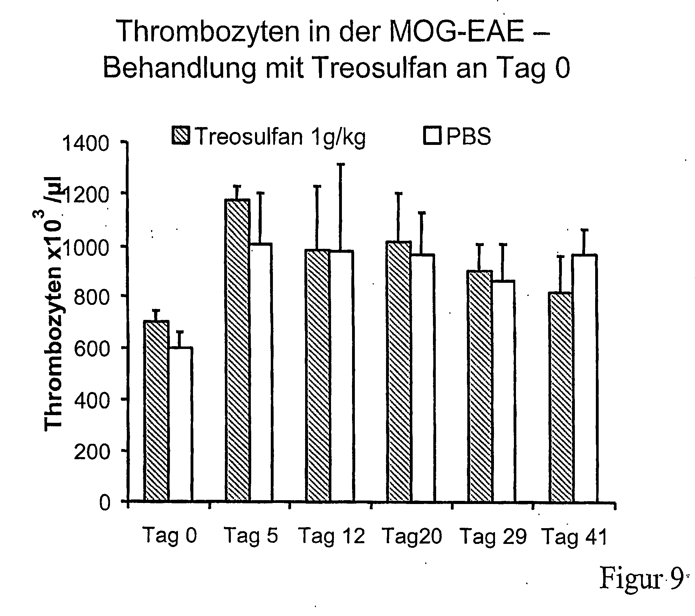

- Fig. 9:: Thrombozyten in der MOG-EAE-Behandlung mit Treosulfan an Tag 0.

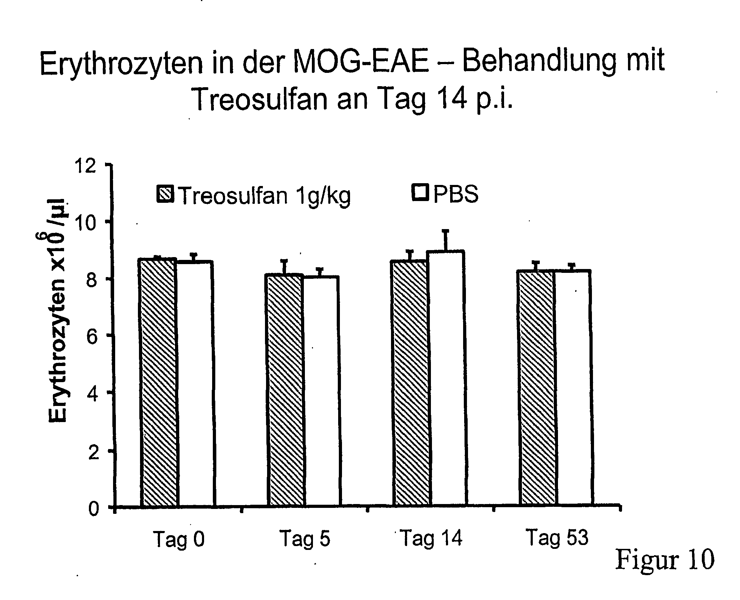

- Fig. 10:: Erythrozyten in der MOG-EAE-Behandlung mit Treosulfan an Tag 14 p.i.

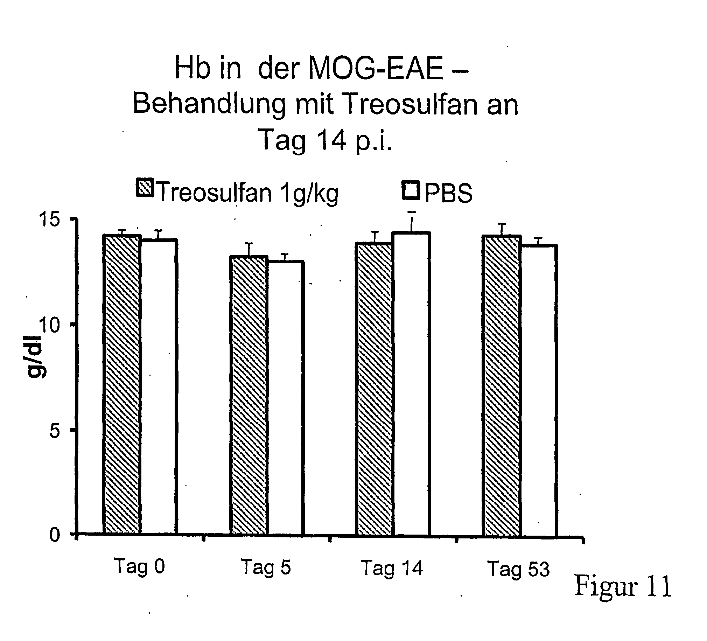

- Fig. 11:: Hämoglobin (Hb) in der MOG-EAE-Behandlung mit Treosulfan an Tag 14 p.i.

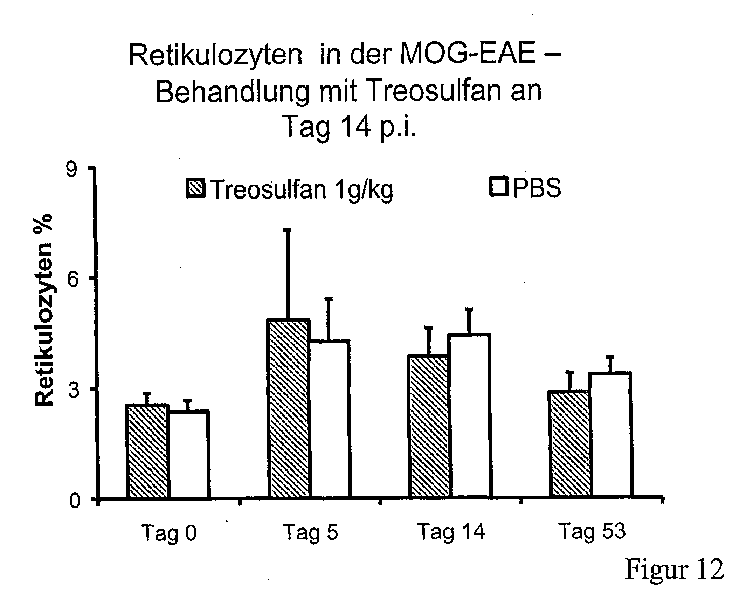

- Fig. 12:: Retikulozyten in der MOG-EAE-Behandlung mit Treosulfan an Tag 14 p.i.

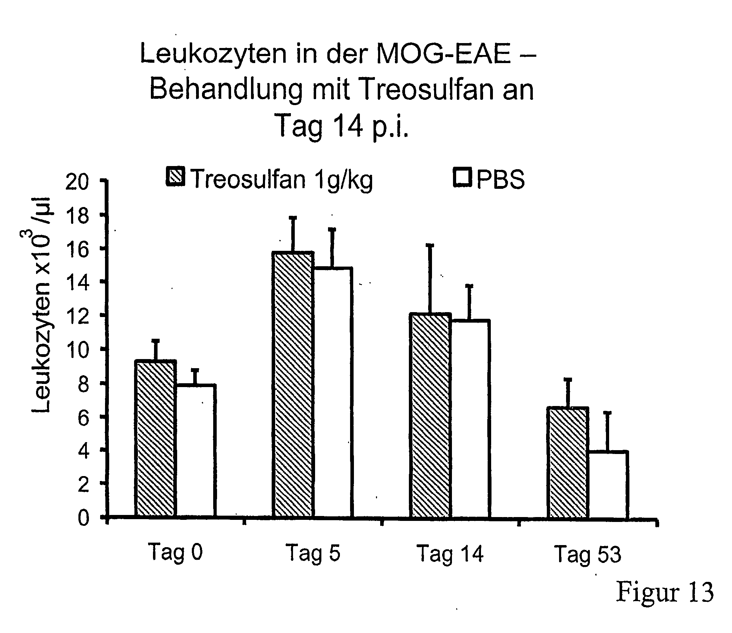

- Fig. 13:: Leukozyten in der MOG-EAE-Behandlung mit Treosulfan an Tag 14 p.i.

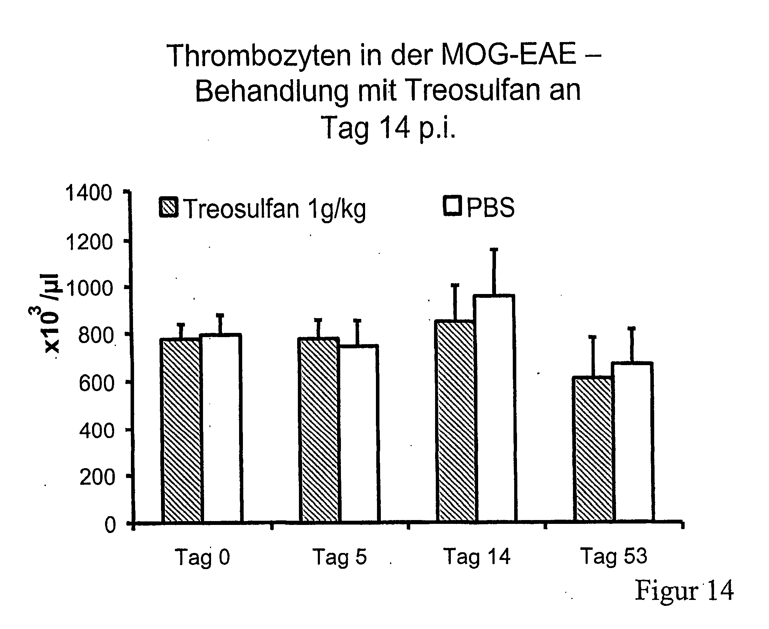

- Fig. 14:: Thrombozyten in der MOG-EAE-Behandlung mit Treosulfan an Tag 14 p.i.

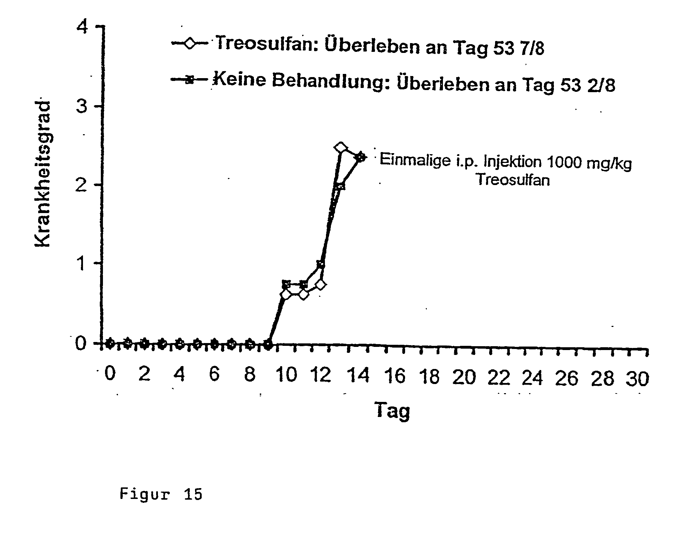

- Fig. 15:: Einmalige i.p. Injektion 1000 mg/kg Treosulfan.
- Fig. 16:: Quantitative PCR für IFN- -Behandlung mit Treosulfan an Tag 0.
- Fig. 17:: Quantitative PCR für IL-12-Behandlung mit Treosulfan an Tag 0.

## Patentansprüche

1. Verwendung von Treosulfan und/oder Derivaten davon zur Herstellung eines Arzneimittels, zur Behandlung von Multipler Sklerose (MS).

2. Verwendung nach Anspruch 1, **dadurch gekennzeichnet, daß** die MS eine schubförmig-remittierende, primär progressive oder sekundär progressive MS ist.

3. Verwendung nach den Ansprüchen 1 und 2, **dadurch gekennzeichnet, daß** das Derivat Busulfan, Dimethylbusulfan, Pentasulfan oder Hepsulfam ist.

4. Verwendung nach den Ansprüchen 1 bis 3, , **dadurch gekennzeichnet, daß** das Treosulfan oder das Derivat davon in einer zur Verabreichung von 1 bis 10 g Treosulfan und/oder Treosulfan-Derivat pro m² Körperoberfläche geeigneten Form vorliegt.

5. Verwendung nach Anspruch 4 zur Verabreichung von 3 bis 9 oder 5 bis 8 g Treosulfan und/oder Treosulfan-Derivat pro m² Körperoberfläche.

6. Verwendung nach den Ansprüchen 1 bis 5, , **dadurch gekennzeichnet, daß** man Treosulfan und/oder ein Derivat davon in Kombination mit einer oder mehreren immunmodulatorisch wirksamen Substanzen verwendet.

7. Verwendung nach Anspruch 6, **dadurch gekennzeichnet, daß** die immunmodulatorisch wirksame Substanz Interferon- und/oder Glatriameracetat ist.

8. Verwendung nach den Ansprüchen 1 bis 7 in Form einer Infusionslösung oder einer oralen Formulierung.

## Claims

1. Use of treosulfan and/or derivatives thereof for producing a pharmaceutical for treating multiple sclerosis (MS).

2. Use according to Claim 1, **characterized in that** the MS is an intermittently remitting, primarily progressive or secondarily progressive MS.

3. Use according to Claims 1 and 2, **characterized in that** the derivative is busulfan, dimethylbusulfan, pentasulfan or hepsulfam.

4. Use according to Claims 1 to 3, **characterized in that** the treosulfan, or the derivative thereof, is present in a form which is suitable for administering from 1 to 10 g of treosulfan and/or treosulfan derivative per m² of body surface.

5. Use according to Claim 4 for administering from 3 to 9 or from 5 to 8 g of treosulfan and/or treosulfan derivative per m² of body surface.

6. Use according to Claims 1 to 5, **characterized in that** treosulfan and/or a derivative thereof is/are used in combination with one or more substances having an immunomodulatory effect.

7. Use according to Claim 6, **characterized in that** the substance having an immunomodulatory effect is interferon acetate and/or glatiramer acetate.

8. Use according to Claims 1 to 7 in the form of an infusion solution or of an oral formulation.

## Revendications

1. Utilisation de tréosulfan et/ou de ses dérivés, pour la fabrication d'un médicament destiné au traitement de la sclérose en plaques (SEP).

2. Utilisation selon la revendication 1, **caractérisée en ce que** la SEP est une SEP récidivante par poussées, progressive primaire ou progressive secondaire.

3. Utilisation selon les revendications 1 et 2, **caractérisée en ce que** le dérivé est le busulfan, le diméthylbusulfan, le pentasulfan ou l'hepsulfam.

4. Utilisation selon les revendications 1 à 3, **caractérisée en ce que** le tréosulfan ou le dérivé de tréosulfan se trouve sous une forme appropriée à l'administration de 1 à 10 g de tréosulfan et/ou de dérivé de tréosulfan par m² de surface corporelle.

5. Utilisation selon la revendication 4, pour l'administration de 3 à 9 ou 5 à 8 g de tréosulfan et/ou de dérivé de tréosulfan par m² de surface corporelle.

6. Utilisation selon les revendications 1 à 5, **caractérisée en ce qu'**on utilise du tréosulfan et/ou un dérivé de tréosulfan en association avec une ou plusieurs substances à activité immunoniodulatrice.

7. Utilisation selon la revendication 6, **caractérisée en ce que** la substance immunomodulatrice est l'interféron et/ou l'acétate de glatiramer.

8. Utilisation selon les revendications 1 à 7, sous forme d'une solution pour perfusion ou d'une composition orale.
